# EUROPEAN PATENT APPLICATION

(11) **EP 1 990 427 A1**
(43) Date of publication of application: **12.11.2008**
(21) Application number: 07715139.7
(22) Date of filing: 23.02.2007
(51) Int. Cl.: C12Q 1/68, A61K 38/00, A61K 45/00, A61P 1/10, A61P 1/14, A61P 43/00, C12Q 1/02, C07K 14/47, C12N 1/15, C12N 1/19, C12N 1/21, C12N 5/10, C12N 15/09, G01N 33/566

(54) **NPY Y4 AGONIST AS THERAPEUTIC AGENT FOR DISEASE ACCOMPANIED BY INTESTINAL TRACT DYSFUNCTION**

(30) Priority: 24.02.2006 JP 2006048438
(71) Applicant: BANYU PHARMACEUTICAL CO., LTD., Tokyo 102-8667 (JP)
(72) Inventor: SUZUKI, Jun, Tsukuba-shi, Ibaraki, 300-2611 (JP); MORIYA, Ryuichi, Tsukuba-shi, Ibaraki, 300-2611 (JP); HIROSE, Hiroyasu, Tsukuba-shi, Ibaraki, 300-2611 (JP); KANNO, Tetsuya, Tsukuba-shi, Ibaraki, 300-2611 (JP); KANATANI, Akio, Tsukuba-shi, Ibaraki, 300-2611 (JP)
(74) Representative: Buchan, Gavin MacNicol
(86) International application number: PCT/JP2007/053989
(87) International publication number: WO 2007/100071

(57) **Abstract**

The present invention has its object to provide an agent capable of ameliorating the intestinal tract dysfunction through a novel mechanism of action. It also has its object to provide a method for the evaluation of a compound, including the screening of the agent. A disease accompanied by intestinal tract dysfunction can be ameliorated or treated by using an NPY Y4 agonist as a therapeutic agent. A compound capable of ameliorating or treating a disease accompanied by intestinal tract dysfunction can be evaluated or screened by evaluating a compound targeting to an NPY Y4 receptor.

SEQUENCE LISTING

<110>  BANYU Pharmaceutical co., ltd.

<120>  NPY Y4 agonist as a therapeutic agent for functional disorder of bowel

<130>  BY0166

<160>  6

<170>  PatentIn version 3.1

<210>  1
<211>  1180
<212>  DNA
<213>  Homo sapiens

<220>
<221>  CDS
<222>  (28)..(1155)
<223>

<400>  1
gagtcctgga atcttttcac atccact atg aac acc tct cac ctc ctg gcc ttg      54
                              Met Asn Thr Ser His Leu Leu Ala Leu
                              1               5

ctg ctc cca aaa tct cca caa ggt gaa aac aga agc aaa ccc ctg ggc      102
Leu Leu Pro Lys Ser Pro Gln Gly Glu Asn Arg Ser Lys Pro Leu Gly
10                  15                  20                  25

acc cca tac aac ttc tct gaa cat tgc cag gat tcc gtg gac gtg atg      150
Thr Pro Tyr Asn Phe Ser Glu His Cys Gln Asp Ser Val Asp Val Met
                30                  35                  40

gtc ttc atc gtc act tcc tac agc att gag act gtc gtg ggg gtc ctg      198
Val Phe Ile Val Thr Ser Tyr Ser Ile Glu Thr Val Val Gly Val Leu
            45                  50                  55

ggt aac ctc tgc ctg atg tgt gtg act gtg agg cag aag gag aaa gcc      246
Gly Asn Leu Cys Leu Met Cys Val Thr Val Arg Gln Lys Glu Lys Ala
        60                  65                  70

aac gtg acc aac ctg ctt atc gcc aac ctg gcc ttc tct gac ttc ctc      294
Asn Val Thr Asn Leu Leu Ile Ala Asn Leu Ala Phe Ser Asp Phe Leu
    75                  80                  85

atg tgc ctc ctc tgc cag ccg ctg acc gcc gtc tac acc atc atg gac      342
Met Cys Leu Leu Cys Gln Pro Leu Thr Ala Val Tyr Thr Ile Met Asp
90                  95                  100                 105

tac tgg atc ttt gga gag acc ctc tgc aag atg tcg gcc ttc atc cag      390
Tyr Trp Ile Phe Gly Glu Thr Leu Cys Lys Met Ser Ala Phe Ile Gln
                110                 115                 120

tgc atg tcg gtg acg gtc tcc atc ctc tcg ctc gtc ctc gtg gcc ctg      438
Cys Met Ser Val Thr Val Ser Ile Leu Ser Leu Val Leu Val Ala Leu
            125                 130                 135

gag agg cat cag ctc atc atc aac cca aca ggc tgg aag ccc agc atc      486
Glu Arg His Gln Leu Ile Ile Asn Pro Thr Gly Trp Lys Pro Ser Ile
        140                 145                 150

tca cag gcc tac ctg ggg att gtg ctc atc tgg gtc att gcc tgt gtc      534
Ser Gln Ala Tyr Leu Gly Ile Val Leu Ile Trp Val Ile Ala Cys Val
    155                 160                 165

ctc tcc ctg ccc ttc ctg gcc aac agc atc ctg gag aat gtc ttc cac      582

Leu Ser Leu Pro Phe Leu Ala Asn Ser Ile Leu Glu Asn Val Phe His
170                 175                 180                 185

aag aac cac tcc aag gct ctg gag ttc ctg gca gat aag gtg gtc tgt      630
Lys Asn His Ser Lys Ala Leu Glu Phe Leu Ala Asp Lys Val Val Cys
                190                 195                 200

acc gag tcc tgg cca ctg gct cac cac cgc acc atc tac acc acc ttc      678
Thr Glu Ser Trp Pro Leu Ala His His Arg Thr Ile Tyr Thr Thr Phe
            205                 210                 215

ctg ctc ctc ttc cag tac tgc ctc cca ctg ggc ttc atc ctg gtc tgt      726
Leu Leu Leu Phe Gln Tyr Cys Leu Pro Leu Gly Phe Ile Leu Val Cys
        220                 225                 230

tat gca cgc atc tac cgg cgc ctg cag agg cag ggg cgc gtg ttt cac      774
Tyr Ala Arg Ile Tyr Arg Arg Leu Gln Arg Gln Gly Arg Val Phe His
    235                 240                 245

aag ggc acc tac agc ttg cga gct ggg cac atg aag cag gtc aat gtg      822
Lys Gly Thr Tyr Ser Leu Arg Ala Gly His Met Lys Gln Val Asn Val
250                 255                 260                 265

gtg ctg gtg gtg atg gtg gtg gcc ttt gcc gtg ctc tgg ctg cct ctg      870
Val Leu Val Val Met Val Val Ala Phe Ala Val Leu Trp Leu Pro Leu
                270                 275                 280

cat gtg ttc aac agc ctg gaa gac tgg cac cat gag gcc atc ccc atc      918
His Val Phe Asn Ser Leu Glu Asp Trp His His Glu Ala Ile Pro Ile
            285                 290                 295

tgc cac ggg aac ctc atc ttc tta gtg tgc cac ttg ctt gcc atg gcc      966
Cys His Gly Asn Leu Ile Phe Leu Val Cys His Leu Leu Ala Met Ala
        300                 305                 310

tcc acc tgc gtc aac cca ttc atc tat ggc ttt ctc aac acc aac ttc      1014
Ser Thr Cys Val Asn Pro Phe Ile Tyr Gly Phe Leu Asn Thr Asn Phe
    315                 320                 325

aag aag gag atc aag gcc ctg gtg ctg act tgc cag cag agc gcc ccc      1062
Lys Lys Glu Ile Lys Ala Leu Val Leu Thr Cys Gln Gln Ser Ala Pro
330                 335                 340                 345

ctg gag gag tcg gag cat ctg ccc ctg tcc aca gta cat acg gaa gtc      1110
Leu Glu Glu Ser Glu His Leu Pro Leu Ser Thr Val His Thr Glu Val
                350                 355                 360

tcc aaa ggg tcc ctg agg cta agt ggc agg tcc aat ccc att taa          1155
Ser Lys Gly Ser Leu Arg Leu Ser Gly Arg Ser Asn Pro Ile
            365                 370                 375

ccaggtctag gtcttctccc tgcca                                          1180


<210>  2
<211>  375
<212>  PRT
<213>  Homo sapiens

<400>  2

Met Asn Thr Ser His Leu Leu Ala Leu Leu Leu Pro Lys Ser Pro Gln
1               5                   10                  15


Gly Glu Asn Arg Ser Lys Pro Leu Gly Thr Pro Tyr Asn Phe Ser Glu
            20                  25                  30

His Cys Gln Asp Ser Val Asp Val Met Val Phe Ile Val Thr Ser Tyr
        35                  40                  45

Ser Ile Glu Thr Val Val Gly Val Leu Gly Asn Leu Cys Leu Met Cys
    50                  55                  60

Val Thr Val Arg Gln Lys Glu Lys Ala Asn Val Thr Asn Leu Leu Ile
65                  70                  75                  80

Ala Asn Leu Ala Phe Ser Asp Phe Leu Met Cys Leu Leu Cys Gln Pro
                85                  90                  95

Leu Thr Ala Val Tyr Thr Ile Met Asp Tyr Trp Ile Phe Gly Glu Thr
            100                 105                 110

Leu Cys Lys Met Ser Ala Phe Ile Gln Cys Met Ser Val Thr Val Ser
        115                 120                 125

Ile Leu Ser Leu Val Leu Val Ala Leu Glu Arg His Gln Leu Ile Ile
    130                 135                 140

Asn Pro Thr Gly Trp Lys Pro Ser Ile Ser Gln Ala Tyr Leu Gly Ile
145                 150                 155                 160

Val Leu Ile Trp Val Ile Ala Cys Val Leu Ser Leu Pro Phe Leu Ala
                165                 170                 175

Asn Ser Ile Leu Glu Asn Val Phe His Lys Asn His Ser Lys Ala Leu
            180                 185                 190

Glu Phe Leu Ala Asp Lys Val Val Cys Thr Glu Ser Trp Pro Leu Ala
        195                 200                 205

His His Arg Thr Ile Tyr Thr Thr Phe Leu Leu Leu Phe Gln Tyr Cys
    210                 215                 220

Leu Pro Leu Gly Phe Ile Leu Val Cys Tyr Ala Arg Ile Tyr Arg Arg
225                 230                 235                 240

Leu Gln Arg Gln Gly Arg Val Phe His Lys Gly Thr Tyr Ser Leu Arg
                245                 250                 255

Ala Gly His Met Lys Gln Val Asn Val Val Leu Val Val Met Val Val
            260                 265                 270

Ala Phe Ala Val Leu Trp Leu Pro Leu His Val Phe Asn Ser Leu Glu
        275                 280                 285

Asp Trp His His Glu Ala Ile Pro Ile Cys His Gly Asn Leu Ile Phe
    290                 295                 300

Leu Val Cys His Leu Leu Ala Met Ala Ser Thr Cys Val Asn Pro Phe
305                 310                 315                 320

Ile Tyr Gly Phe Leu Asn Thr Asn Phe Lys Lys Glu Ile Lys Ala Leu
                325                 330                 335

Val Leu Thr Cys Gln Gln Ser Ala Pro Leu Glu Glu Ser Glu His Leu
            340                 345                 350

Pro Leu Ser Thr Val His Thr Glu Val Ser Lys Gly Ser Leu Arg Leu
        355                 360                 365

Ser Gly Arg Ser Asn Pro Ile
    370                 375

<210>  3
<211>  36
<212>  PRT
<213>  Homo sapiens

<400>  3

Ala Pro Leu Glu Pro Val Tyr Pro Gly Asp Asn Ala Thr Pro Glu Gln
1               5                   10                  15

Met Ala Gln Tyr Ala Ala Asp Leu Arg Arg Tyr Ile Asn Met Leu Thr
            20                  25                  30

Arg Pro Arg Tyr
        35

<210>  4
<211>  36
<212>  PRT
<213>  Bovine

<400>  4

Ala Pro Leu Glu Pro Glu Tyr Pro Gly Asp Asn Ala Thr Pro Glu Gln
1               5                   10                  15

Met Ala Gln Tyr Ala Ala Glu Leu Arg Arg Tyr Ile Asn Met Leu Thr
            20                  25                  30

Arg Pro Arg Tyr
        35

<210>  5
<211>  36
<212>  PRT
<213>  Rat

<400>  5

Ala Pro Leu Glu Pro Met Tyr Pro Gly Asp Tyr Ala Thr His Glu Gln
1               5                   10                  15

Arg Ala Gln Tyr Glu Thr Gln Leu Arg Arg Tyr Ile Asn Thr Leu Thr
            20                  25                  30

Arg Pro Arg Tyr
        35

<210>  6
<211>  36
<212>  PRT
<213>  mouse

<400>  6

Ala Pro Leu Glu Pro Met Tyr Pro Gly Asp Tyr Ala Thr Pro Glu Gln
1               5                   10                  15

Met Ala Gln Tyr Glu Thr Gln Leu Arg Arg Tyr Ile Asn Thr Leu Thr
            20                  25                  30

Arg Pro Arg Tyr
        35

## Description

### Technical Field

The present invention relates to a method for evaluating a compound effective in the diagnosis or treatment of a disease accompanied by intestinal tract dysfunction, which targets an NPY Y4 agonist. Further, the present invention relates to a novel use of an NPY Y4 agonist aiming at the diagnosis or treatment of a disease accompanied by intestinal tract dysfunction.

### Background Art

NPY (neuropeptide Y) is a peptide consisting of 36 amino acids, and was isolated from the porcine brain by Tatemoto et al. in 1982 (Non-patent document 1). NPY is widely distributed in the central nervous system and peripheral nervous system, and is in charge of a variety of functions in vivo as one of the most abundant peptides in the nervous systems.

That is, NPY functions as an orexigenic substance in the central nervous system, and also promotes markedly the fat accumulation via the mediation of the secretion of various hormones or the action of the nervous systems. For example, it is known that continuous intracerebroventricular administration of NPY induces obesity and insulin resistance on the basis of such actions. It is also known that NPY has central actions such as regulation of depression, anxiety, schizophrenia, pain, dementia and circadian rhythm, etc. Further, NPY coexists with norepinephrine at the sympathetic nerve terminals in the peripheral nervous system, and is related to the tonicity of the sympathetic nervous system. It is known that peripheral administration of NPY causes vasoconstriction and also enhances the actions of other vasoconstrictive substances such as norepinephrine.

NPY has a wide variety of pharmacological actions via the receptors partially shared by its analogs, peptide YY (hereinafter referred to as PYY) and a pancreatic polypeptide (hereinafter referred to as PP). These pharmacological actions of NPY are known to be induced by the action of at least five types of receptors such as NPY Y1 to Y5 either solely or through their mutual interactions (Non-patent document 2).

On the other hand, with regard to an NPY Y4 receptor, a human NPY Y4 receptor gene has already been isolated (Non-patent documents 3 and 4, and Patent document 1, Accession No. U35232 (SEQ ID NOs: 1 and 2)). The human NPY Y4 receptor has been isolated by screening a human genomic library using an NPY Y1 receptor cDNA as a probe, and has been revealed to have a seven transmembrane structure and to show a homology of 42% with the Y1 receptor at the amino acid level. Further, it has been revealed that the NPY Y4 receptor exhibits an affinity for PP as well as NPY, increases the intracellular calcium concentration and shows an inhibitory effect on cAMP production like an NPY Y1 receptor or Y2 receptor. Further, it has been known that the NPY Y4 receptor is mainly expressed in the prostate gland, colon, pancreas and small intestine.

It has been reported that in NPY Y4 receptor knockout mice, the body weight and the total amount of white adipose tissue decrease and the plasma PP level increases (Non-patent document 5).
(Patent document 1) WO 95/17906
(Non-patent document 1) Nature, vol. 296, p. 659, 1982
(Non-patent document 2) Trends in Neuroscience, vol. 20, p. 294, 1997
(Non-patent document 3) The Journal of Biological Chemistry, vol. 270, p. 26762, 1995
(Non-patent document 4) The Journal of Biological Chemistry, vol. 270, p. 29123, 1995
(Non-patent document 5) Genes and Development, vol. 16, p. 1077, 2002

### Disclosure of the Invention

However, the present situation is that there is no knowledge of the relationship between an NPY Y4 receptor and intestinal movement, and as an agent for ameliorating intestinal tract dysfunction (for example, constipation), an agent based on a novel mechanism with less side effects has been demanded.

The present invention has been made in view of the above-mentioned problem of the prior art, and has its object to provide an agent capable of ameliorating intestinal tract dysfunction through a novel mechanism of action. It also has its object to provide a method for evaluating a compound, including screening of such an agent.

The present inventors made intensive studies in order to achieve the above objects, and as a result, they found that an NPY Y4 agonist acts on the intestinal tract and ameliorates intestinal tract dysfunction through the action, and thus completed the present invention.

That is, the method for evaluating a compound of the present invention is a method for evaluating a compound to be used for the diagnosis or treatment of a disease caused by intestinal tract dysfunction, and is characterized by comprising the steps of: preparing a cell expressing NPY Y4 by introducing an NPY Y4 gene; bringing a test compound into contact with the cell; and detecting a specific binding of the test compound to the NPY Y4. According to the evaluation method, a compound which acts on an NPY Y4 receptor and treats or ameliorates intestinal tract dysfunction can be evaluated or screened.

Further, the method for evaluating a compound of the present invention is a method for evaluating a compound to be used for the diagnosis or treatment of a disease caused by intestinal tract dysfunction, and is characterized by comprising the steps of: preparing a cell expressing NPY Y4 by introducing an NPY Y4 gene; bringing a test compound into contact with the cell; measuring the activity of an intracellular signal transmitter induced by the contact; and comparing the above-mentioned activity with the activity of the intracellular signal transmitter in the absence of contact with the test compound. According to the evaluation method, a compound which has a function of regulating the intracellular signal transduction mediated by an NPY Y4 receptor by acting on the NPY Y4 receptor and treats or ameliorates intestinal tract dysfunction can be evaluated or screened.

Further, the method for evaluating a compound of the present invention is a method for evaluating a compound to be used for the diagnosis or treatment of a disease caused by intestinal tract dysfunction, and is characterized by comprising the steps of: preparing a cell expressing NPY Y4 by introducing an NPY Y4 gene; bringing a test compound into contact with the cell; measuring the expression level of the NPY Y4 or an intracellular signal transmitter mediated by the NPY Y4; and selecting the test compound which increased or decreased the expression level of the NPY Y4 or the intracellular signal transmitter in comparison with that in the absence of contact with the test compound. According to the evaluation method, a compound which has a function of regulating the intracellular signal transduction mediated by an NPY Y4 receptor by acting on the NPY Y4 receptor and treats or ameliorates intestinal tract dysfunction can be evaluated or screened.

Further, the evaluation method of the present invention is a method for evaluating a compound to be used for the diagnosis or treatment of a disease caused by intestinal tract dysfunction, and is characterized by comprising the steps of: bringing a test compound into contact with NPY Y4; and detecting a change in the activity of NPY Y4 caused by the contact. According to the evaluation method, a compound which has a function of regulating the function of NPY Y4 by interacting with an NPY Y4 protein and treats or ameliorates intestinal tract dysfunction can be evaluated or screened.

Further, the method for evaluating a compound of the present invention is characterized by using an NPY Y4 gene-modified nonhuman mammal or a tissue or a cell derived from the mammal. By evaluating a test compound using the animal, the in vivo function or behavior of a compound which specifically binds to NPY Y4 can be observed and a compound can be more accurately evaluated.

Further, in the above-mentioned five embodiments of the method for evaluating a compound, the compound is preferably an NPY Y4 agonist.

Further, the therapeutic agent for a disease caused by intestinal tract dysfunction of the present invention is characterized by comprising an NPY Y4 agonist as an active ingredient. Here, the NPY Y4 agonist is preferably a pancreatic polypeptide.

Further, the kit for screening a therapeutic agent for a disease accompanied by intestinal tract dysfunction of the present invention is characterized by comprising NPY Y4 or a partial peptide thereof. By using the kit, a therapeutic agent for a disease accompanied by intestinal tract dysfunction can be simply and promptly screened or evaluated.

Further, the kit for screening a therapeutic agent for a disease accompanying intestinal tract dysfunction of the present invention is characterized by comprising a cell expressing a protein having an amino acid sequence identical or substantially identical to that of NPY Y4 or a partial peptide thereof. By using the kit, a therapeutic agent for a disease accompanied by intestinal tract dysfunction can be simply and promptly screened or evaluated without preparing a cell to be used for the screening or evaluation of a compound.

That is, according to the present invention, an agent capable of ameliorating intestinal tract dysfunction (for example, constipation) based on a novel mechanism of action can be provided. Further, a method for evaluating a compound having an action of ameliorating intestinal tract dysfunction, including screening of such an agent, and a kit capable of simply and promptly performing the evaluation can be provided. Further, according to the method for evaluating a compound of the present invention, a therapeutic agent, which has a small effect on the small intestine and a high selectivity for the large intestine (particularly the ileum), can be obtained. Accordingly, a therapeutic agent, which not only shows a marked effect of ameliorating constipation but also has less side effects on other body parts or organs can be obtained.

### Brief Description of the Drawings

Fig. 1 is a view showing an in vivo relationship between the dose of PP and the amount of excreted feces over time.
Fig. 2 is a view showing an in vivo relationship between the dose of PP and the total amount of excreted feces for 4 hours after administration.
Fig. 3 is a view showing a relationship between the dose of PP and an intestinal tension in the ileum, proximal colon or distal colon.
Fig. 4 is a view showing a relationship between the administration of PP and the number of amplitudes of intestine in the case of the ileum, proximal colon and distal colon.
Fig. 5 is a view showing the amount of excreted feces for 4 hours after administration in the case where PP (1 mg/kg) was administered to mouse models of constipation.
Fig. 6 is a view showing the amount of excreted feces for 2 hours after administration in the case where PP was administered to NPY Y4 receptor knockout mice.
Fig. 7 is a view showing a difference in the intestinal tension when PP was administered in the ileum, proximal colon or distal colon isolated from an NPY Y4 receptor knockout mouse and a wild-type mouse.

### Best Mode for Carrying Out the Invention

Hereinafter, a preferred embodiment of the present invention will be described in detail.

The term "NPY Y4" receptor according to the present invention means a molecule having a seven transmembrane structure that functions as a receptor for NPY as described above. Further, the species from which the NPY Y4 receptor is derived is not particularly limited, and examples thereof include humans, monkeys, mice, rats, dogs and rabbits. In particular, because a subject to which the NPY Y4 agonist of the present invention is administered, or a subject to be evaluated in the evaluation of a compound is a human, it is preferably human NPY Y4.

Further, in the NPY Y4 gene according to the present invention, a gene having substitution, deletion, addition or insertion of one or more nucleotides is also included as long as it has a physiological function equivalent to that of the NPY Y4 gene and encodes a protein that functions as an NPY receptor. Here, the gene is not particularly limited in terms of its sequence as long as it is a gene encoding such a protein. However, it preferably has a homology of 50% or more, more preferably 70% or more, further more preferably 80% or more, and particularly preferably 90% or more (for example, 91, 92, 93, 94, 95, 96, 97, 98, 99% or more).

Further, in the NPY Y4 gene according to the present invention, a nucleic acid which is hybridized to the NPY Y4 gene under a stringent condition is also included. Here, the phrase "which is hybridized under a stringent condition" means that two nucleic acid fragments are hybridized to each other under the hybridization condition described in Molecular Cloning: A Laboratory Manual 2nd Edition, Cold Spring Harbor (1989), 9.47-9.62 and 11.45-11.61. More specifically, for example, a condition in which washing is carried out at 50°C with 2.0 x SSC after hybridization is carried out at about 45°C with 6.0 x SSC can be exemplified. In order to select the stringency, the salt concentration in the washing step can be selected from about 2.0 x SSC at 50°C as a low stringency to about 0.2 x SSC at 50°C as a high stringency. Further, the temperature in the washing step can be raised from room temperature of about 22°C as a low stringency condition to about 65°C as a high stringency condition.

Further, the disease caused by intestinal tract dysfunction according to the present invention is not particularly limited, and examples thereof include constipation, acute appendicitis, irritable bowel syndrome with constipation and ischemic colitis. Further, specific examples of such constipation include transient simple constipation, syndromic constipation, spastic constipation, flaccid constipation, rectal constipation and drug-induced constipation.

### (1) Evaluation of compound

A compound that acts on NPY Y4 can be evaluated using an NPY Y4 gene or protein. Examples of the method of detecting an action against NPY Y4 include a method of detecting a specific binding of a test compound to the receptor, a method of detecting the expression level of the gene which is changed by the contact with a test compound and a method of detecting the activity of intracellular signal transduction mediated by NPY Y4 induced by the contact. Hereinafter, these methods will be described in turn.

First, the method for evaluating a test compound by detecting a specific binding of a test compound to the receptor will be described.

A first embodiment of the method for evaluating a compound of the present invention is characterized by comprising the steps of: preparing a cell expressing NPY Y4 by introducing an NPY Y4 gene; bringing a test compound into contact with the cell; and detecting a specific binding of the test compound to the NPY Y4.

Further, the cell expressing an NPY Y4 gene can be prepared by a method known to a person skilled in the art, and a specific method is not particularly limited, and for example, the following method can be employed. That is, the cell is prepared by cloning an NPY Y4 gene or a nucleic acid consisting of a part thereof into an expression vector containing a suitable promoter and a transcriptional regulatory element, and introducing the vector with the cloned nucleic acid into a host cell. Here, the vector is not particularly limited as long as it is a vector that can be used as an expression vector, and examples thereof include pCMV-Tag, pcDNA3.1, pBlueBacHis2, pCI-neo, pcDNAI, pMC1neo, pXT1, pSG5, pEF1/V5-HisB, per2.1, pET11, λgt11 and pCR3.1.

Subsequently, the expression vector into which the NPY Y4 gene or the nucleic acid consisting of a part thereof has been introduced is transfected into a host cell. Such a host cell is not particularly limited as long as it is a host cell commonly used in the expression of a gene, and may be any of an animal cell, an insect cell, a plant cell, and a microorganism. Specific examples thereof include COS1, COS7, CHO, NIH/3T3, 293, Raji, CV11, C1271, MRC-5, CPAE, HeLa, 293T and Sf9. Further, the method of transfecting the expression vector into the host cell is not particularly limited as long as it is a known method, and specific examples thereof include electroporation, a calcium phosphate method, a DEAE-dextran method, a lipofection method and a gene gun method.

The test compound is not particularly limited, and examples thereof include single compounds such as natural compounds, organic compounds, inorganic compounds, proteins and peptides, and compound libraries, expression products of gene libraries, cell extracts, cell culture supernatants, fermentation products of microorganisms, marine organism extracts, plant extracts, prokaryotic cell extracts, eukaryotic single cell extracts, animal cell extracts and the like. The above-mentioned test compound can be used by appropriately labeling if necessary. As the labeling, for example, radiolabeling, fluorescent labeling and the like can be exemplified. Further, in place of the above-mentioned test compounds, a mixture obtained by mixing plural types of these test compounds can be used.

Further, in the present invention, the "contact" is carried out according to the state of NPY Y4. For example, if NPY Y4 is in a state of being expressed on a cell surface or in a state of being expressed in a cell extract, the contact can be carried out by adding a test compound to a cell culture medium or the cell extract, respectively. In the case where the test compound is a protein, for example, a vector containing a DNA encoding the protein is transfected into a cell expressing NPY Y4. Alternatively, the contact can be carried out by adding the vector to a cell extract in which NPY Y4 is expressed. Further, for example, a two-hybrid method using yeast, an animal cell or the like can also be used.

Specifically, in order to screen a compound which changes the binding of a test compound to NPY Y4, a receptor preparation is prepared by suspending a cell containing NPY Y4 or a membrane fraction of the cell in a buffer suitable for screening. The buffer may be any as long as it is a buffer which does not inhibit the binding of a ligand to a receptor such as a phosphate buffer or a Tris-HCl buffer (pH 4 to 10, preferably pH 6 to 8). Also, for the purpose of decreasing non-specific binding, a surfactant such as CHAPS, digitonin or deoxycholate can be added to the buffer. Also, for the purpose of suppressing the degradation of the receptor of the present invention due to a protease, a protease inhibitor such as PMSF, leupeptin or pepstatin can be added to the buffer. To 0.01 to 10 ml of the solution, a given amount (for example, 5000 to 500000 cpm) of a labeled test compound is added, and at the same time, 10⁻¹⁰ to 10⁻⁴ M of a testing compound is allowed to coexist. In order to determine the non-specific binding amount, a reaction tube to which a large excess amount of the unlabeled ligand of the present invention has been added is also prepared. The reaction is carried out at 0°C to 50°C, preferably at 4°C to 37°C for 20 minutes to 24 hours, preferably for 30 minutes to 3 hours. After the reaction, the reaction solution is filtered through a glass fiber filter paper or the like, the filter is washed with an appropriate amount of the same buffer. Then, the radioactivity remaining on the glass fiber filter paper is measured using a liquid scintillation counter or a γ-counter.

In the first embodiment, subsequently, the binding of NPY Y4 to a test compound is detected. The detection method is not particularly limited. The binding of a receptor expressed on a cell surface to a test compound can be detected by, for example, a label attached to the bound compound (for example, detection of binding amount by radioactivity or fluorescence intensity) as described above, and other than this, it can be detected by using signal transduction into a cell caused by binding of the test compound to the receptor on the cell surface (for example, G protein activation, change in the concentration of cAMP or Ca²⁺ (FLIPR (fluorometric imaging plate reader) or the like can be employed), phospholipase C activation, pH change, or receptor internalization) as an index.

Specifically, the intracellular signal transduction mediated by NPY Y4 (for example, an activity of promoting or suppressing arachidonic acid release, acetylcholine release, intracellular Ca²⁺ release, intracellular cAMP production, intracellular cAMP production suppression, intracellular cGMP production, inositol phosphate (IP) production, change in cell membrane potential, phosphorylation of intracellular proteins, activation of c-fos, pH reduction, GTPγS binding activity, activation of cAMP-dependent protein kinase, activation of cGMP-dependent protein kinase, activation of phospholipid-dependent protein kinase, activation of microtubule-associated protein kinase (MAP kinase) or the like) can be measured using a known method or a commercially available assay kit.

Specifically, first, a cell containing NPY Y4 is cultured on a multiwell-plate or the like. Upon implementation of screening, the culture medium is changed to a freshly prepared medium or an appropriate buffer showing no toxicity to the cell beforehand, and a test compound is added thereto and the mixture is incubated for a given time. Then, the cell is extracted or the supernatant is recovered, and the resulting products are determined according to the respective methods. In the case where the production of a substance (for example, inositol triphosphate or the like) which is an index for the cell stimulating activity is difficult to be assayed due to a degrading enzyme contained in the cell, an inhibitor of the degrading enzyme may be added to perform an assay. Further, with regard to the activity of cAMP production suppression, it can be detected as an action of suppressing the production against a cell in which basic production amount of the cell has been increased using forskolin or the like.

In the first embodiment, subsequently, a test compound which binds to NPY Y4 is selected. In the selected compound, a compound which promotes or suppresses the activity of NPY Y4 and a compound which increases or decreases the expression of NPY Y4 are included, and such compounds have an action of promoting or suppressing intestinal movement such as peristaltic movement. When a compound which functions as an agonist of NPY Y4 is selected from these compounds, the compound can be used as an agent effective in preventing or treating a disease accompanying intestinal tract dysfunction.

Further, a second embodiment of the method for evaluating a compound of the present invention is characterized by comprising the steps of: preparing a cell expressing NPY Y4 by introducing an NPY Y4 gene; bringing a test compound into contact with the cell; measuring the activity of an intracellular signal transmitter induced by the contact; and comparing the above-mentioned activity with the activity of the intracellular signal transmitter in the absence of contact with the test compound.

In the second embodiment, first, a test compound is brought into contact with a cell expressing NPY Y4.

The cell expressing NPY Y4 can be prepared as follows. That is, the preparation of a cell expressing an NPY Y4 protein by introducing an NPY Y4 gene can be carried out by a method known to a person skilled in the art, and for example, the cell is prepared by cloning an NPY Y4 gene or a nucleic acid consisting of a part thereof into an expression vector containing a suitable promoter and a transcriptional regulatory element, and introducing the vector with the cloned nucleic acid into a host cell. Here, the vector is not particularly limited as long as it is a vector that can be used as an expression vector, and examples thereof include pCMV-Tag, pcDNA3.1, pBlueBacHis2, pCI-neo, pcDNAI, pMC1neo, pXT1, pSG5, pEF1/V5-HisB, pCR2.1, pET11, λgt11 and pCR3.1.

Subsequently, the expression vector into which the NPY Y4 gene or the nucleic acid consisting of a part thereof has been introduced is transfected into a host cell. Such a host cell is not particularly limited as long as it is a host cell commonly used in the expression of a gene, and may be any of an animal cell, an insect cell, a plant cell, and a microorganism. Examples thereof include SW480, DLD-1, CCD-18Co, CCD-841CoN, COS1, COS7, CHO, NIH/3T3, 293, Raji, CV11, C1271, MRC-5, CPAE, HeLa, 293T and Sf9. Further, the method of transfecting the expression vector into the host cell is not particularly limited as long as it is a known method, and examples thereof include electroporation, a calcium phosphate method, a DEAE-dextran method and a lipofection method.

Further, as the test compound to be used in the second embodiment, a compound similar to that used in the first embodiment can be used.

Subsequently, a test compound is brought into contact with the thus prepared cell expressing NPY Y4. The method of such contact is not particularly limited, and for example, if NPY Y4 is in a state of being expressed in a cell (including on a cell membrane) or in a state of being expressed in a cell extract, the contact can be carried out by adding a test sample to a cell culture medium or the cell extract, respectively. In the case where the test sample is a protein, for example, a vector containing a DNA encoding the protein is transfected into a cell expressing NPY Y4. Alternatively, the contact can also be carried out by adding the vector to a cell extract in which NPY Y4 is expressed.

Subsequently, the activity of an intracellular signal transmitter induced by the contact between NPY Y4 and the test compound is measured. Specifically, for example, the activity of a molecule which is in charge of the intracellular signal transduction mediated by NPY Y4 such as phospholipase C (PLC) or inositol triphosphate (IP3) is measured.

In the case of IP3, the level of intracellular inositol triphosphate is increased by an NPY Y4 agonist. By utilizing this phenomenon, the level of inositol triphosphate is measured in the case where a test compound is brought into contact with a cell which has expressed an NPY Y4 receptor and in the case of the absence of contact in the presence of labeled inositol. When the level increased, the test compound can be judged to have an activity as an NPY Y4 agonist. Here, the measurement of the level of inositol triphosphate can be carried out by a known method. More specifically, for example, a cell which has expressed NPY Y4 is plated on a 96-well plate, and cultured for one day. Thereafter, the cell is cultured in a medium supplemented with myo-[³H]inositol for one day, followed by washing the cell with a medium which does not contain labeled inositol. After a test compound is added thereto, 10% perchloric acid is added thereto to stop the reaction. The reaction solution is neutralized with 1.5 M potassium hydroxide and a 60 mM HEPES solution, and then passed through a column packed with 0.5 ml of AG1 x 8 resin (Bio-Rad). After washing with 5 mM sodium tetraborate and 60 mM ammonium formate, the radioactivity eluted with 1 M ammonium formate and 0.1 M formic acid is measured using a liquid scintillation counter, whereby the level of IP3 can be measured and calculated.

Further, the activity can also be measured by using the binding activity between the test compound and NPY Y4 as an index. The method is not particularly limited, and specific examples thereof include a method in which the binding activity is measured by measuring the affinity of the test compound for a membrane on which NPY Y4 has been immobilized. The test compound to be used here can be labeled with a radioisotope or the like so as to facilitate the detection thereof. Further, as the method of detecting the binding activity, a method of detecting a compound that binds to NPY Y4 in a manner competitive with a ligand labeled with a radioisotope can also be exemplified. In the case where such a method is used, the test compound does not need to be labeled.

As described above, in the case where as a result of detecting a compound by the method for evaluating a compound of the present invention, the binding activity in the presence of a test compound has a lower value than the binding activity in the absence of the test compound (control), the test compound is judged to have an activity of inhibiting the binding between the NPY Y4 according to the present invention and the ligand. Such compounds include compounds which have an activity of inducing signal transduction into a cell through binding to the receptor (agonists), compounds which do not have such an activity (antagonists) and the like. An agonist has a physiological activity similar to that of the ligand for the receptor and its analogs, while an antagonist inhibits the physiological activity of the ligand for the receptor and its analogs. Thus, such agonists and antagonists have a function of regulating the progression of signal transduction mediated by the NPY Y4 according to the present invention and are useful as a pharmaceutical composition for the treatment or the like of a disease caused by an abnormality in such a signal transduction system or the like.

Further, a third embodiment of the method for evaluating a compound of the present invention is a method for evaluating a compound to be used for the diagnosis or treatment of a disease caused by intestinal tract dysfunction, and is characterized by comprising the steps of: preparing a cell expressing NPY Y4 by introducing an NPY Y4 gene; bringing a test compound into contact with the cell; measuring the expression level of the NPY Y4 or an intracellular signal transmitter mediated by the NPY Y4; and selecting the test compound which increased or decreased the expression level of the NPY Y4 or the intracellular signal transmitter in comparison with that in the absence of contact with the test compound.

In the third embodiment, first, a test compound is brought into contact with a cell expressing NPY Y4.

The cell expressing NPY Y4 can be prepared as follows. That is, the preparation of a cell expressing an NPY Y4 protein by introducing an NPY Y4 gene can be carried out by a method known to a person skilled in the art, and for example, the cell is prepared by cloning an NPY Y4 gene or a nucleic acid consisting of a part thereof into an expression vector containing a suitable promoter and a transcriptional regulatory element, and introducing the vector with the cloned nucleic acid into a host cell. Here, the vector is not particularly limited as long as it is a vector that can be used as an expression vector, and examples thereof include pCMV-Tag, pcDNA3.1, pBlueBacHis2, pCI-neo, pcDNAI, pMC1neo, pXT1, pSG5, pEF1/V5-HisB, pCR2.1, pET11, λgt11 and pCR3.1.

Subsequently, the expression vector into which the NPY Y4 gene or the nucleic acid consisting of a part thereof has been introduced is transfected into a host cell. Such a host cell is not particularly limited as long as it is a host cell commonly used in the expression of a gene, and may be any of an animal cell, an insect cell, a plant cell, and a microorganism. Examples thereof include SW480, DLD-1, CCD-18Co, CCD-841CoN, COS1, COS7, CHO, NIH/3T3, 293, Raji, CV11, C1271, MRC-5, CPAE, HeLa, 293T and Sf9. Further, the method of transfecting the expression vector into the host cell is not particularly limited as long as it is a known method, and specific examples thereof include electroporation, a calcium phosphate method, a DEAE-dextran method and a lipofection method.

Further, as the test compound to be used in the third embodiment, a compound similar to that used in the first embodiment can be used.

In the present invention, the "contact" can be carried out as follows. If NPY Y4 is in a state of being expressed in a cell or in a state of being expressed in a cell extract, the contact can be carried out by adding a test compound to a cell culture medium or the cell extract, respectively. In the case where the test compound is a protein, for example, a vector containing a DNA encoding the protein is transfected into a cell expressing NPY Y4. Alternatively, the contact can be carried out by adding the vector to a cell extract in which NPY Y4 is expressed. Further, for example, a two-hybrid method using yeast, an animal cell or the like can also be used.

In the third embodiment, subsequently, the expression level of NPY Y4 is measured. Here, the term "expression level" as used herein refers to the absolute amount or relative amount of a transcription product of a gene encoding a protein in the signal transduction pathway mediated by NPY Y4. In this case, in the gene, both DNA and mRNA are included. Further, in the case where the detection target for expression is a protein, the term "expression level" refers to the absolute amount or relative amount of a protein in the signal transduction pathway mediated by NPY Y4. Further, in the case where the activity of a molecule involved in signal transduction is used as an index, the method of measuring the activity is not particularly limited, and a suitable method may be selected depending on the type of a molecule to be measured.

The measurement of the expression level of NPY Y4 can be carried out by a method known to a person skilled in the art. For example, mRNA of an NPY Y4 gene is extracted according to a common procedure, and the transcription level of the NPY Y4 gene can be measured by performing a Northern hybridization method or an RT-PCR method using the resulting mRNA as a template. Further, by using a DNA array technique, the expression level of the NPY Y4 gene can also be measured.

Further, by recovering fractions containing NPY Y4 encoded by an NPY Y4 gene according to a common procedure and detecting the expression of NPY Y4 by an electrophoresis method such as SDS-PAGE, the translation level of the gene can also be measured. Further, by performing a Western blotting method using an antibody against NPY Y4 and detecting the expression of NPY Y4, the gene can also be measured at the translation level. Here, the antibody to be used for the detection of NPY Y4 is not particularly limited as long as it is a detectable antibody, and may be either a monoclonal antibody or a polyclonal antibody. The antibody can be prepared by a method known to a person skilled in the art. Specifically, for example, in the case of a polyclonal antibody, it can be prepared as follows. That is, a rabbit or the like is immunized with NPY Y4 or a recombinant protein expressed in a microorganism such as E. coli as a fusion protein between NPY Y4 and GST or a partial peptide thereof, and the serum of the rabbit is obtained, followed by purification through, for example, ammonium sulfate precipitation, a protein A column, a protein G column, ion exchange chromatography, an affinity column coupled with NPY Y4 or the like, whereby a polyclonal antibody can be prepared. Further, in the case of a monoclonal antibody, for example, a small animal such as a mouse is immunized with NPY Y4 or a partial peptide thereof, the spleen is isolated from the mouse and ground, and cells are separated. Then, the resulting cells are fused with mouse myeloma cells using a reagent such as polyethylene glycol. Among the thus obtained fused cells (hybridomas), a clone which produces an antibody binding to NPY Y4 is selected. Then, the resulting hybridoma is transplanted into the abdominal cavity of a mouse, the ascites is collected from the mouse, and the resulting monoclonal antibody is purified through, for example, ammonium sulfate precipitation, a protein A column, a protein G column, ion exchange chromatography, an affinity column coupled with NPY Y4 or the like, whereby a monoclonal antibody can be prepared.

In the third embodiment, subsequently, a test compound which decreased or increased the expression level of the NPY Y4 in comparison with that in the absence of contact with the test compound is selected. In the selected compound, a compound which decreases or increases the expression of the NPY Y4 is included. A compound which decreases the expression functions as an NPY Y4 antagonist, and a compound which enhances the expression functions as an NPY Y4 agonist. When a compound which functions as an agonist of NPY Y4 is selected from these compounds, the compound can be used as an agent effective in preventing or treating a disease accompanied by intestinal tract dysfunction.

Further, a forth embodiment of the method for evaluating a compound of the present invention is a method for evaluating a compound to be used for the diagnosis or treatment of a disease caused by intestinal tract dysfunction, and is characterized by comprising the steps of: bringing a test compound into contact with NPY Y4; and detecting a change in the activity of NPY Y4 caused by the contact.

The purification of NPY Y4 can be carried out by a known method. Further, examples of the cell expressing NPY Y4 include a cell expressing endogenous NPY Y4 and a cell expressing exogenous NPY Y4. Examples of the cell expressing endogenous NPY Y4 include a cultured cell and the like, however, it is not limited thereto. The cultured cell is not particularly limited, and for example, a commercially available cultured cell can be used. Further, the species from which the cell expressing endogenous NYP Y4 is derived is not particularly limited, and examples thereof include humans, mice, rats, monkeys, guinea pigs, ferrets and the like. Further, the cell expressing exogenous NPY Y4 can be prepared by, for example, introducing a vector containing a DNA encoding NPY Y4 into a cell. The introduction of the vector into a cell can be carried out by a common method such as a calcium phosphate method, electroporation, a lipofectamine method or a microinjection method.

Further, examples of the cell extract in which NPY Y4 is expressed include those obtained by adding a vector containing a DNA encoding NPY Y4 to a cell extract contained in an in vitro transcription/translation system. The in vitro transcription/translation system is not particularly limited, and a commercially available in vitro transcription/translation kit or the like can be used.

Further, as the test compound to be used in the fourth embodiment, a compound similar to that used in the first embodiment can be used.

In the fourth embodiment, the method of such contact is not particularly limited, and specific examples thereof include a method in which the contact is carried out by mixing in a solution such as a buffer (a phosphate buffer or the like), and a method in which an NPY Y4 protein is immobilized on a membrane and the protein is brought into contact with a test compound on the membrane.

Subsequently, a change in the activity of NPY Y4 caused by the contact is detected.

The method of measuring the activity of a protein may be appropriately set depending on the nature of a protein to be used, and specific examples thereof include a method in which the binding activity of a ligand for NPY Y4 is used as an index.

The method in which the binding activity of a ligand is used as an index is not particularly limited, and specific examples thereof include a method in which the binding activity is determined by measuring the affinity of a test compound for a membrane on which NPY Y4 has been immobilized. The test compound to be used here may be labeled with a radioisotope or the like so as to facilitate the detection. Further, as the method of detecting the binding activity, a method of detecting a compound that binds to NPY Y4 in a manner competitive with a ligand labeled with a radioisotope can also be exemplified. In the case where such a method is used, the test compound does not need to be labeled.

As described above, in the case where as a result of detecting a compound by the method for evaluating a compound of the present invention, the binding activity in the presence of a test compound has a lower value than the binding activity in the absence of the test compound (control), the test compound is judged to have an activity of inhibiting the binding between the NPY Y4 according to the present invention and the ligand. Such compounds include compounds which have an activity of inducing signal transduction into a cell through binding to the receptor (agonists), compounds which do not have such an activity (antagonists) and the like. An agonist has a physiological activity similar to that of the ligand for the receptor and its analogs, while an antagonist inhibits the physiological activity of the ligand for the receptor and its analogs. Thus, such agonists and antagonists have a function of regulating the progression of signal transduction mediated by the NPY Y4 according to the present invention and are useful as a pharmaceutical composition for the treatment and the like of a disease caused by an abnormality in such a signal transduction system or the like. When a compound which functions as an agonist of NPY Y4 is selected from these compounds, the compound can be used as an agent effective in preventing or treating a disease accompanied by intestinal tract dysfunction.

A fifth embodiment of the method for evaluating a compound of the present invention is characterized by using an NPY Y4 gene-modified nonhuman mammal or a tissue or a cell derived from the mammal.

The genetically modified nonhuman mammal to be used in the evaluation method is not particularly limited in terms of its method for suppressing NPY Y4 and can be prepared according to a known method. For example, a genetically modified mouse can be produced as follows. First, a DNA containing an exon region of an NPY Y4 gene is isolated from a mouse, and a part or the whole of the NPY Y4 gene sequence is deleted or another gene is inserted or substituted by a genetic engineering technique. In general, an appropriate marker gene is inserted into a part or the whole of the NPY Y4 gene sequence, whereby a targeting vector is constructed. As the marker gene to be inserted, an antibiotic resistance gene such as a neomycin resistance gene or a hygromycin resistance gene; or a reporter gene such as a β-galactosidase gene (lacz), a chloramphenicol acetyltransferase gene (cat), a luciferase gene or a GFP (green fluorescent protein) gene is preferred. In the case where an antibiotic resistance gene is inserted, a cell line in which homologous recombination has occurred can be selected only by culturing cells with a medium containing the antibiotic. Further, in order to perform more efficient selection, it is also possible to ligate a thymidine kinase gene or the like to the targeting vector. By doing this, a cell line in which nonhomologous recombination has occurred can be eliminated. Further, in the case where the function of exon is disrupted by inserting a reporter gene, the reporter gene is preferably inserted such that the gene is expressed under the control of the promoter of NPY Y4. Further, examples of the vector to be used in the preparation of the targeting vector include pBR322, pBR325, pUC12, pUB110, pTB5, pSH19, pSH15 and pK0.

The thus obtained targeting vector is introduced into an ES cell line of a mouse or the like by an electroporation method or the like, and a cell line in which homologous recombination has occurred is selected. Specifically, the targeting vector is introduced into a nonhuman mammalian ES cell or a nonhuman animal egg cell by a known method (an electroporation method, a microinjection method, a calcium phosphate method, a lipofection method, a particle gun method or the like) and substituting the inactivated NPY Y4 gene sequence contained in the targeting vector with the NPY Y4 gene on the chromosome of the nonhuman animal ES cell or nonhuman animal egg cell by homologous recombination, whereby the selection can be carried out.

An NPY Y4 gene knockout cell can be determined by a Southern hybridization analysis using a DNA sequence on the NPY Y4 gene or in the vicinity thereof as a probe or an analysis through a PCR method using a DNA sequence on the targeting vector and a DNA sequence of a region in the vicinity of the NPY Y4 gene as primers.

In the case where a nonhuman animal ES cell is used, a cell line in which the NPY Y4 gene has been inactivated by homologous recombination is cloned, and the resulting cloned cell is injected into a nonhuman animal embryo or blastocyst at an appropriate stage in the early stage of embryogenesis such as the 8-cell stage. Alternatively, a cluster of ES cells in which the NPY Y4 gene has been inactivated is sandwiched between two 8-cell stage embryos. The resulting chimeric embryo is transplanted into the uterus of a pseudopregnant nonhuman animal.

The thus produced animal is a chimeric animal composed of both cells: a cell having a normal NPY Y4 gene locus; and a cell having an artificially mutated NPY Y4 gene locus. In the case where some germ cells of the chimeric animal have a mutated NPY Y4 gene locus, an individual whose entire tissue is composed of cells having an artificially mutated MGAT2 gene locus can be obtained by selection from a group of offsprings obtained by crossing between such a chimeric animal and a normal animal, by, for example, determination of the coat color or the like. The thus obtained individual is generally an NPY Y4 heterozygous animal, and an NPY Y4 homozygous animal can be obtained from offsprings of the intercross between NPY Y4 heterozygous animals.

In the case where an egg cell is used, for example, a transgenic nonhuman animal in which a targeting vector has been introduced into the chromosome by injecting a gene solution into the nucleus of an egg cell through a microinjection method can be obtained. By comparing the thus obtained transgenic nonhuman animals, an animal which has a mutation at the NPY Y4 gene locus by homologous recombination can be obtained by selection.

The thus produced NPY Y4 gene knockout nonhuman mammal is deficient in the NPY Y4 receptor in the body. Accordingly, even when an NPY Y4 agonist or antagonist is administered to such a mammal, a physiological function due to the agonist or antagonist is never exhibited. The present inventors have found that when an NPY Y4 agonist is administered to a mouse, the amount of excreted feces increases and the tension of the intestinal tract is elevated, however, this phenomenon is not observed in such an NPY Y4 gene knockout mouse. That is, by administering a compound evaluated or screened according to the first to the fourth embodiments of the method for evaluating a compound described above to an NPY Y4 knockout nonhuman mammal or evaluating a compound using a tissue of the animal, it becomes possible to confirm that the compound specifically binds to NPY Y4 and acts thereon.

Specifically, an NPY Y4 gene knockout mouse and a wild-type mouse are prepared, and a test compound is administered to both mice. The administration method is not particularly limited, however, a method through oral administration or subcutaneous administration can be exemplified. After administration, feces are collected and the weight thereof is measured, whereby the amount of excreted feces due to the administration of the test compound is determined. When the amount of excreted feces in the case of the wild-type mouse is significantly larger than in the case of the NPY Y4 gene knockout mouse, the test compound is a compound specifically acts on NPY Y4, therefore, the compound can be judged to have an NPY Y4 agonistic activity.

Further, the action of a test compound can be studied by using the intestinal tracts isolated from an NPY Y4 gene knockout mouse and a wild-type mouse. For example, the intestinal tract isolated from a mouse is cut into a suitable length and is suspended under a desired resting tension in Krebs-Henseleit solution. By applying a test compound to the suspended intestinal tract and measuring a change in the tension, a compound that acts specifically on NPY Y4 can be selected. Here, by changing the site (for example, ileum, colon and the like) of the intestinal tract to be isolated and subjecting it to the test, a compound that functions in a site specific manner can be selected.

Further, according to the method for evaluating a compound of the present invention, a substance which promotes or inhibits the intracellular signal transduction after binding of a test compound to NPY Y4 can be screened. That is, by evaluating multiple test compounds by the above-mentioned method, a compound that functions as an agonist or an antagonist can be selected. If, as a result of such selection, in comparison with the change in the intracellular signal transduction in the case where the ligand or its analog is allowed to act in the absence of the test compound, the change is suppressed, the test compound is judged to be a compound that inhibits the intracellular signal transduction after binding of the test compound to NPY Y4. On the contrary, if the test compound enhances the intracellular signal transduction, the compound is judged to be a compound that promotes the intracellular signal transduction after binding of the test compound to NPY Y4. A compound selected by such a screening method is effective in the treatment of a disease accompanied by intestinal tract dysfunction or the alleviation of the symptom, and above all, a compound that functions as an NPY Y4 agonist is effective in the treatment of a disease accompanied by intestinal tract dysfunction or the alleviation of the symptom. Further, according to the method for evaluating a compound of the present invention, a therapeutic agent, which has a small effect on the small intestine and has a high selectivity for the large intestine (particularly the ileum), can be obtained. Accordingly, a therapeutic agent, which not only shows a marked effect of ameliorating constipation but also has less side effects on other body parts or organs can be obtained.

### (2) NPY Y4 agonist and therapeutic agent containing the agonist as active ingredient

The "NPY Y4 agonist" as used herein means a substance causing intracellular signal transduction mediated by an NPY Y4 receptor and is also referred to as an NPY Y4 stimulating agent. Further, the NPY Y4 agonist according to the present invention is not particularly limited in terms of its molecular species as long as it is a molecule that exhibits an affinity for an NPY Y4 receptor and functions as an agonist. Examples of such a molecule include low molecular compounds, proteins, and peptides. The low molecular compound is also not particularly limited in terms of its type, and specific examples thereof include natural compounds, organic compounds and inorganic compounds. Further, the peptide is also not particularly limited in terms of its type, and specific examples thereof include NPY analogs such as PP.

Here, PP (human PP: SEQ ID NO: 3, bovine PP: SEQ ID NO: 4, rat PP: SEQ ID NO: 5, mouse PP: SEQ ID NO: 6) refers to a peptide having a homology of about 50% with NPY at the amino acid level, and is called the NPY family together with PYY (peptide YY) (J. Biol. Chem., vol. 250, p. 9369, 1975). PP is a peptide consisting of 36 amino acids similar to NPY, and is expressed mainly in the pancreas. When PYY is administered to a mouse, the amount of food intake is suppressed, therefore, PYY has been suggested to function as a regulatory factor for appetite or food intake in the body (J. Clin, Endocr. Metab., vol. 88, p. 3989, 2003). Further, there has also been a report that suggests some relationship between the amount of secreted PP and diarrhea, however, the causal relationship between them from the functional point of view has not been elucidated yet (Gut, vol. 24, p. 665, 1983; Inter. J. of Gastrointet. Cancer, vol. 32, p. 153, 2002; Pancreas, vol. 29, p. 83, 2004).

In PP according to the present invention, a peptide which has a function as an NPY Y4 agonist and has substitution, deletion, addition or insertion of one or more amino acids in any one of the amino acid sequences represented by SEQ ID NOs: 2 to 5 is also included. The number of amino acids to be substituted, deleted, added or inserted in such a case is not particularly limited as long as the peptide has a function as an NPY Y4 agonist. However, the number of amino acids is preferably 1 to 10, more preferably 1 to 8, further more preferably 1 to 5, and particularly preferably 1 to 3.

Further, the NPY Y4 agonist of the present invention can be synthesized by a synthetic method known to a person skilled in the art according to the type of compound if it is a compound. Further, if the compound is derived from a natural product, it can be purified from the natural product by a given extraction method. Further, if the agonist is a peptide, it can be synthesized by a method known to a person skilled in the art.

In the case where the NPY Y4 agonist of the present invention is used as a therapeutic agent for humans or other animals, it is possible to administer any of these substances by formulating it into a preparation by a known pharmaceutical method other than the direct administration of the substance as such to a patient. For example, it can be used orally as a tablet, if necessary coated with a sugar, a capsule, an elixir or a microcapsule, or parenterally in the form of an injection of a sterile solution or a suspension with water or a pharmaceutically acceptable liquid other than water. For example, it is possible to formulate the substance into a preparation by appropriately combining a pharmacologically acceptable carrier or medium, specifically sterile water, saline, a vegetable oil, an emulsifying agent, a suspending agent, a surfactant, a stabilizer, a flavoring agent, an excipient, a vehicle, a preservative, a binder or the like, and mixing them in a unit dosage form as required by generally admitted pharmaceutical practice.

As an additive which can be mixed in a tablet or a capsule, for example, a binder such as gelatin, cornstarch, tragacanth gum or gum arabic, an excipient such as crystalline cellulose, a swelling agent such as cornstarch, gelatin or alginic acid, a lubricant such as magnesium stearate, a sweetener such as sucrose, lactose or saccharine, or a flavoring agent such as peppermint, acamono oil or cherry is used. In the case where the preparation unit is in the capsule form, a liquid carrier such as fat and oil may further be added to the above-mentioned materials. A sterile composition for injection can be formulated according to a common pharmaceutical practice using a vehicle such as distilled water for injection.

Examples of an aqueous solution for injection include saline and isotonic solutions containing glucose or other adjuvants (for example, D-sorbitol, D-mannose, D-mannitol and sodium chloride), and may be used in combination with an appropriate solubilizing agent such as an alcohol, specifically ethanol, a polyalcohol such as propylene glycol or polyethylene glycol or a nonionic surfactant such as Polysorbate 80 (TM) or HCO-50.

Examples of an oily liquid include sesame oil and soybean oil, and may be used in combination with a solubilizing agent such as benzyl benzoate or benzyl alcohol. In addition, it may be further mixed with a buffer such as a phosphate buffer or a sodium acetate buffer, a soothing agent such as procaine hydrochloride, a stabilizer such as benzyl alcohol or phenol, or an antioxidant. The thus prepared injectable solution is usually filled into an appropriate ampoule.

The administration thereof to a patient can be carried out intranasally, transbronchially, intramuscularly, percutaneously or orally by a method known to a person skilled in the art as well as by way of, for example, intraarterial injection, intravenous injection, subcutaneous injection or the like. The dose varies depending on the body weight or age of a patient, an administration route or the like, however, a person skilled in the art can appropriately select a suitable dose. Further, if the agonist can be one encoded by a DNA, it is also possible to perform gene therapy by inserting the DNA into a vector for gene therapy. The dose and administration method vary depending on the body weight, age or symptom of a patient or the like, however, a person skilled in the art can appropriately select them.

The dose of the NPY Y4 agonist varies depending on the symptom, however, in the case of oral administration, the dose thereof is considered to be about 0.1 to 100 mg per day, preferably about 1.0 to 50 mg per day, more preferably about 1.0 to 20 mg per day for generally an adult (assuming of 60 kg of body weight).

In the case of parenteral administration, a single dose thereof varies depending also on the subject to be administered, target organ, symptom, or administration method. However, in the case of, for example, an injection, it is considered to be convenient to administer the substance in an amount of generally about 0.01 to 30 mg per day, preferably about 0.1 to 20 mg per day, more preferably about 0.1 to about 10 mg per day by intravenous injection for generally an adult (assuming of 60 kg of body weight).

According to the present invention, a therapeutic agent, which has a small effect on the small intestine and has a high selectivity for the large intestine (particularly the ileum), can be provided. Accordingly, a therapeutic agent, which not only shows a marked effect of ameliorating constipation but also has less side effects on other body parts or organs can be provided.

### (3) Kit for screening therapeutic agent for disease caused by intestinal tract dysfunction

The kit for screening of the present invention is characterized by comprising at least a protein having an amino acid sequence identical or substantially identical to that of NPY Y4 or a partial peptide thereof. The protein or a partial peptide thereof may be labeled.

The partial peptide is not particularly limited in terms of the number of amino acids and its site on NPY Y4 as long as it has a partial sequence of NPY Y4 and a GPCR activity similar to that of NPY Y4.

Further, the kit may further include a reaction buffer, a reaction stop solution, a washing buffer, a container to be used for reaction or washing or the like other than NPY Y4 or a partial peptide thereof.

A method for evaluating a test compound using the kit for screening of the present invention is as follows. That is, after a reaction buffer is added to a reaction container, NPY Y4 or a peptide having a partial sequence thereof contained in the kit and a test compound are added thereto, and a binding reaction between the test compound and NPY Y4 is allowed to proceed. Here, it is preferred that a sample to which a control (negative control and/or positive control) compound in place of the test compound has been added is prepared. The reaction may be stopped with a desired reaction stop solution. After stopping the reaction, by detecting the labeled test compound binding to NPY Y4, the binding activity of the test compound to NPY Y4 can be determined.

Further, another embodiment of the kit for screening of the present invention is characterized by comprising at least a cell expressing a protein having an amino acid sequence identical or substantially identical to that of NPY Y4 or a partial peptide thereof. Here, the protein having an amino acid sequence substantially identical to that of NPY Y4 refers to a protein having a GPCR activity similar to that of NPY Y4 though having substitution, deletion, addition or insertion of one or more amino acids. For example, a protein having a polymorphism which does not affect the activity can be exemplified. Further, the partial peptide thereof is not particularly limited in terms of the number of amino acids and its site on NPY Y4 as long as it has a kinase activity similar to that of NPY Y4.

Also, the kit for screening may further include a reaction buffer, a washing buffer, a ligand that can be used as a control, a container to be used for reaction or washing or the like other than NPY Y4, a protein substantially identical to NPY Y4 or a partial peptide thereof.

A method for evaluating a test compound using the kit for screening of the present invention is as follows. That is, after a cell contained in the kit is inoculated into a cell culture container, a test compound is added thereto and the reaction is allowed to proceed. Here, it is preferred that a sample to which a control (negative control and/or positive control) compound in place of the test compound has been added is prepared. The reaction may be stopped with a desired reaction stop solution, or may be stopped by lowering the temperature of the reaction solution on ice or the like. After stopping the reaction, by detecting the labeled test compound binding to NPY Y4 in the cell or detecting the intracellular signal transduction mediated by NPY Y4, the NPY Y4 agonistic or antagonistic activity of the test compound can be determined.

### (Examples)

Hereinafter, the present invention will be described further specifically with reference to Examples, however, the present invention is not limited to the following Examples. Example 1

### (Effect of mPP, which is an NPY Y4 agonist, on gastrointestinal movement)

It is known that PP, which is an intestinal peptide, shows a high affinity for an NPY Y4 receptor, which is one of the neuropeptide Y receptor subtypes. Also, it is known that the Y4 receptor is present in the lower gastrointestinal tract. Accordingly, by administering mouse PP (mPP) to a mouse, an effect on the movement of the lower gastrointestinal tract of the mouse was studied. That is, how the amount of excreted feces was changed by the administration of mPP was observed, and a change in the gastrointestinal movement was discussed by assuming that an increase in the amount of excreted feces reflects an increase in the lower gastrointestinal tract movement.

To male mice (C57BL/6J, 15 weeks of age), mPP (0.001, 0.01, 0.1 or 1 mg/kg) was subcutaneously administered, and to a vehicle group, only saline (5 mL/kg) was subcutaneously administered. The mice were subjected to fasting at the same time of the subcutaneous administration of saline or mPP. Feces were collected at 1, 2 and 4 hours after the administration, and the weight of the feces was measured. A significant difference between groups was analyzed using an analysis of variance and the Dunnett's test.

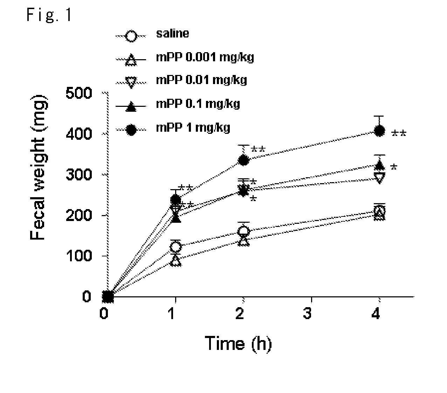

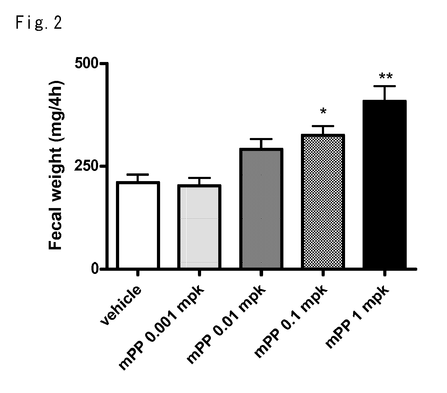

As shown in Figs. 1 and 2, mPP exhibited an action of increasing defecation in a dose dependent manner. The cumulative amounts of excreted feces for 4 hours after administration of mPP were significant at a dose of 0.1 mg/kg or more compared with the vehicle group, and 211 ± 18 mg/4 h, 325 ± 22 mg/4 h (P < 0.05), and 408 ± 36 mg/4 h (P < 0.01) in the vehicle group, group administered with 0.1 mg/kg of mPP and group administered with 1 mg/kg of mPP, respectively. In this connection, in the drawings, ** indicates P < 0.01, and * indicates P < 0.05 (comparison with the vehicle group).

Since an increase in the amount of secreted feces of mice by subcutaneous administration of mPP was observed, an effect of mPP on promoting the lower gastrointestinal tract movement was shown. Accordingly, mPP was considered to be useful as a novel and effective therapeutic agent which ameliorates dysfunction of lower gastrointestinal movement such as constipation and irritable bowel syndrome (IBS) with constipation.

### Example 2

### (Effect of NPY Y4 agonist on spontaneous contraction of the colon isolated from mouse)

In Example 1, an increase in the amount of excreted feces was induced by applying 1 mg/kg of mPP, which is an NPY Y4 agonist, to a mouse. Therefore, in order to confirm the site of action of mPP, whether or not the action of mPP can be observed at the isolated organ level was studied.

The ileum, proximal colon and distal colon were isolated from a C57BL/6 mouse (8 to 20 weeks of age). The specimens were cut into a length of 1 cm, respectively, and were suspended in the longitudinal muscle direction under a resting tension of 0.3 g in 5 mL of Krebs-Henseleit solution (the composition is shown in Table 1) oxygenated with 95% O₂ and 5% CO₂, while setting the temperature of a bath to 37°C. The tension was measured using an isometric transducer (TB-651, manufactured by Nihon Kohden), and outputs were recorded at 20 points per second using Power Lab (manufactured by ADInstrument).

After equilibration for 1 hour, mPP at 0.01 µM, 0.1 µM and 1 µM was applied and measurement was carried out for 20 minutes. The above-mentioned mPP was prepared such that it became 0.01 mM, 0.1 mM and 1 mM solutions in DMSO, and 5 µL of each of the resulting solutions was applied to 5 mL of an organ bath. The action of mPP was represented by a ratio of an average tension for 20 minutes after the application to an average tension for 20 minutes before the application. The statistical calculation was performed using the paired t-test.

**(Table 1)**

| | mM |
|---|---|
| NaCl | 115.0 |
| KCl | 4.7 |
| CaCl₂ | 2.5 |
| MgCl₂·6H₂O | 1.2 |
| KH₂PO₄ | 1.2 |
| Glucose | 5.6 |
| NaHCO₃ | 25.0 |

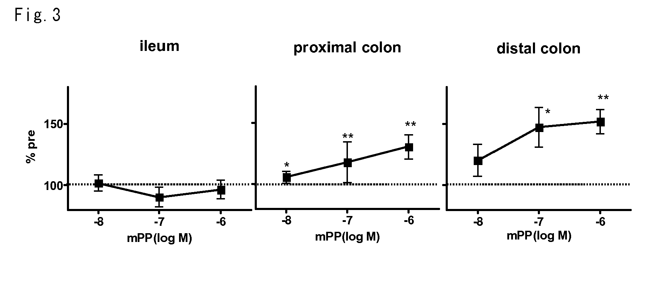

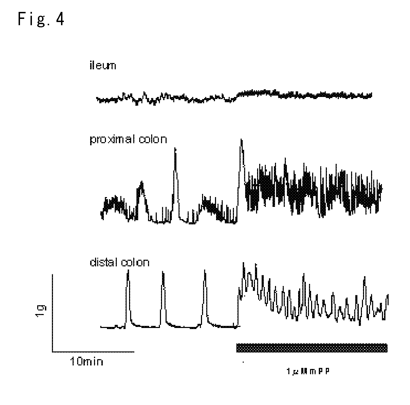

As shown in Fig. 3, in the proximal colon and distal colon of the mouse, mPP increased the tension in a dose dependent manner, and the maximum actions were 131% and 152%, respectively, of those before the application. On the other hand, in the ileum, a significant increase in the tension was not observed. Further, as shown in Fig. 4, in the proximal colon and distal colon, by the application of mPP, not only an increase in the tension, but also an increase in the amplitude of automatism was observed. In this connection, in the drawing, * indicates P < 0.05, and ** indicates P < 0.01 (comparison with the pre-values).

From the above results, in the proximal colon and distal colon of the mouse, an increase in the tension and an increase in the amplitude of automatism by mPP were observed. The colon plays an important role in defecation, and an increase in the movement of the colon is considered to induce diarrhea. The results in this study coincide with the results of an increase in defecation by mPP in vivo. Since the action of mPP was observed also in the isolated tissue, the site of action of mPP was considered to be the peripheral nerve or smooth muscle.

### Example 3

### (Effect of NPY Y4 agonist on mouse models of constipation induced by clonidine)

Since intraperitoneal or subcutaneous administration of mPP to mice increased the amount of excreted feces, whether or not the action of increasing defecation by an NPY Y4 agonist exhibits an ameliorating action in models of constipation induced by clonidine was studied.

To male mice (C57BL/6J, 18 weeks of age), clonidine (0.1 mg/kg) was intraperitoneally administered, and to a vehicle group, only saline was intraperitoneally administered. One hour after the administration of clonidine, saline (a control group) or mPP (1 mg/kg) was subcutaneously administered, and also to a vehicle group, only saline was subcutaneously administered. The mice were subjected to fasting at the same time of the intraperitoneal administration of saline or clonidine. Feces were collected at 1, 2, 3 and 4 hours after the administration, and the weight of the feces was measured. A significant difference between groups was analyzed using an analysis of variance and the Tukey's test.

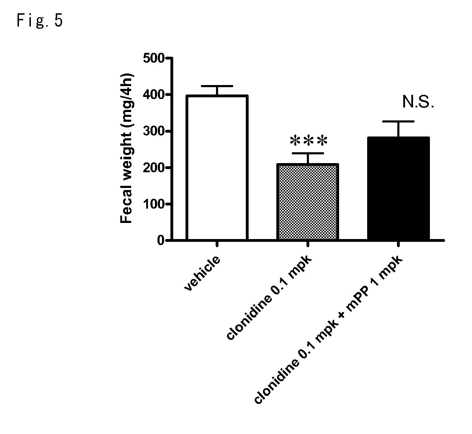

As shown in Fig 5, in the control group, a significant decrease in the amount of excreted feces was observed at 4 hours after the administration compared with the vehicle group (in the drawing, *** indicates P < 0.001). On the other hand, in the group administered with 1 mg/kg of mPP, a significant decrease in the amount of excreted feces was not observed at 4 hours after the administration of clonidine (at 3 hours after the administration of mPP) compared with the vehicle group.

That is, since a post treatment with mPP exhibited an ameliorating action in mouse models of constipation induced by clonidine, an anti-constipation effect of mPP was shown. Accordingly, mPP was considered to be useful as a novel and effective therapeutic agent which ameliorates dysfunction of lower gastrointestinal movement such as constipation and irritable bowel syndrome (IBS) with constipation.

### Example 4

### (Effect of mPP on gastrointestinal movement in NPY Y4 receptor knockout mice)

Since the NPY receptor subtype on which PP acts was unknown, in order to elucidate whether or not an NPY Y4 receptor is involved in the effect of PP on promoting the lower gastrointestinal movement, an effect of PP on the lower gastrointestinal movement in NPY Y4 receptor knockout mice (produced by Deltagen Inc.) was studied.

The following test was carried out using wild-type mice and NPY Y4 receptor knockout mice (male, 23 to 39 weeks of age, n = 10 to 16). mPP (1 mg/kg) or saline (5 mL/kg) was subcutaneously administered to the mice. The mice were subjected to fasting at the same time of the subcutaneous administration. Feces were collected at 1 and 2 hours after the administration, and the weight of the feces was measured. The cumulative amount of feces for 2 hours was used for an analysis, and a significant difference between groups was analyzed using the Student's t-test.

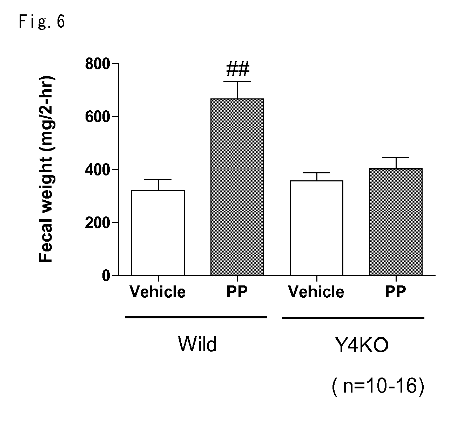

As shown in Fig. 6, mPP exhibited an action of increasing defecation in the wild-type mice (vehicle group: 322 ± 41 mg/2 h, mPP group: 668 ± 64 mg/2 h, P < 0.01). On the other hand, an action of increasing defecation by mPP was not observed in the NPY Y4 receptor knockout mice (vehicle group: 358 ± 31 mg/2 h, mPP group: 403 ± 43 mg/2 h). In this connection, in the drawing, ## indicates P < 0.01 (comparison with the vehicle group).

From the above results, an increase in the amount of excreted feces by the subcutaneous administration of mPP was observed only in the wild-type mice and not observed in the NPY Y4 receptor knockout mice. This indicates that the activation of the NPY Y4 receptor increases the lower gastrointestinal movement. Accordingly, the NPY Y4 receptor agonist was considered to be useful as a novel and effective therapeutic agent which ameliorates dysfunction of lower gastrointestinal movement such as constipation and irritable bowel syndrome with constipation.

### Example 5

### (Effect of NPY Y4 agonist on spontaneous contraction of the colon isolated from NPY Y4 receptor knockout mouse)

In the proximal colon and distal colon of a C57BL/6 mouse, mPP, which is an NPY Y4 agonist, increased the tension. Therefore, in order to confirm whether or not the action of mPP is mediated by an NPY Y4 receptor, a study was carried out using an NPY Y4 receptor knockout mouse.

The ileum, proximal colon and distal colon were isolated from an NPY Y4 receptor knockout mouse and a wild-type mouse (32 to 37 weeks of age). The specimens were cut into a length of 1 cm, respectively, and were suspended in the longitudinal muscle direction under a resting tension of 0.3 g in 5 mL of Krebs-Henseleit solution oxygenated with 95% O₂ and 5% CO₂, while setting the temperature of a bath to 37°C. The tension was measured using an isometric transducer (TB-651, manufactured by Nihon Kohden), and outputs were recorded at 20 points per second using Power Lab (ADInstrument).

After equilibration for 1 hour, 1 µM mPP was applied and measurement was carried out for 20 minutes. The above-mentioned mPP was prepared such that it became a 1 mM solution in DMSO, and 5 µL of the resulting solution was applied to 5 mL of an organ bath. The action of mPP was represented by a ratio of an average tension for 20 minutes after the application to an average tension for 20 minutes before the application. The statistical calculation was performed using the Student's t-test or paired t-test.

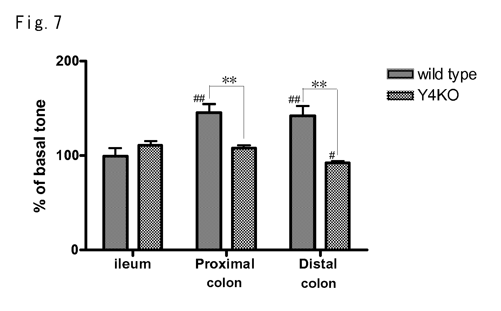

As shown in Fig. 7, in the proximal colon and distal colon, a significant difference in the change in the tension after the application of 1 µM mPP was observed between the wild-type mouse and the NPY Y4 receptor knockout mouse. Further, an increase in the tension observed in the wild-type mouse was not observed in the NPY Y4 receptor knockout mouse. On the other hand, in the ileum, a difference was not observed between them. In this connection, in the drawing, # indicates P < 0.05, and ## indicates P < 0.01 (comparison with the pre-values); * indicates P < 0.05, and * indicates P < 0.01 (comparison between the NPY Y4 receptor knockout mouse and the wild-type mouse).

From the above results, an increase in the tension by mPP observed in the proximal colon and distal colon of the wild-type mouse was not observed in the NPY Y4 receptor knockout mouse, therefore, it was confirmed that an increase in the tension by mPP in the proximal colon and distal colon of the mouse was mediated by the NPY Y4 receptor.

### Industrial Applicability

As described above, according to the NPY Y4 agonist and the method for evaluating a compound of the present invention, an agent capable of ameliorating intestinal tract dysfunction based on a novel mechanism of action can be provided. Further, a method for evaluating a compound having an action of ameliorating intestinal tract dysfunction including screening of such an agent can be provided.

## Claims

1. A method for evaluating a compound to be used for the diagnosis or treatment of a disease caused by intestinal tract dysfunction, **characterized by** comprising the steps of:
preparing a cell expressing NPY Y4 by introducing an NPY Y4 gene;
bringing a test compound into contact with the cell; and
detecting a specific binding of the test compound to the NPY Y4.

2. A method for evaluating a compound to be used for the diagnosis or treatment of a disease caused by intestinal tract dysfunction, **characterized by** comprising the steps of:
preparing a cell expressing NPY Y4 by introducing an NPY Y4 gene;
bringing a test compound into contact with the cell;
measuring the activity of an intracellular signal transmitter induced by the contact; and
comparing said activity with the activity of the intracellular signal transmitter in the absence of contact with the test compound.

3. A method for evaluating a compound to be used for the diagnosis or treatment of a disease caused by intestinal tract dysfunction, **characterized by** comprising the steps of:
preparing a cell expressing NPY Y4 by introducing an NPY Y4 gene;
bringing a test compound into contact with the cell;
measuring the expression level of the NPY Y4 or an intracellular signal transmitter mediated by the NPY Y4; and
selecting the test compound which increased or decreased the expression level of the NPY Y4 or the intracellular signal transmitter in comparison with that in the absence of contact with the test compound.

4. A method for evaluating a compound to be used for the diagnosis or treatment of a disease caused by intestinal tract dysfunction, **characterized by** comprising the steps of:
bringing a test compound into contact with NPY Y4; and
detecting a change in the activity of NPY Y4 caused by the contact.

5. A method for evaluating a compound effective in preventing or treating a disease accompanied by intestinal tract dysfunction, **characterized by** using an NPY Y4 gene knockout nonhuman mammal or a tissue or a cell derived from the mammal.

6. The method for evaluating a compound according to any one of claims 1 to 5, wherein the compound is an NPY Y4 agonist.

7. The method for evaluating a compound according to any one of claims 1 to 6, wherein the disease caused by intestinal tract dysfunction is constipation.

8. A therapeutic agent for a disease caused by intestinal tract dysfunction, comprising an NPY Y4 agonist as an active ingredient.

9. The therapeutic agent according to claim 8, wherein the NPY Y4 agonist is a pancreatic polypeptide.

10. A kit for screening a therapeutic agent for a disease accompanied by intestinal tract dysfunction, comprising NPY Y4 or a partial peptide thereof.

11. A kit for screening a therapeutic agent for a disease accompanied by intestinal tract dysfunction, comprising a cell expressing a protein having an amino acid sequence identical or substantially identical to that of NPY Y4 or a partial peptide thereof.
